# EUROPÄISCHE PATENTANMELDUNG

(11) **EP 3 178 826 A1**
(43) Veröffentlichungstag der Anmeldung: **14.06.2017**
(21) Anmeldenummer: 15198145.3
(22) Anmeldetag: 07.12.2015
(51) Int. Cl.: C07F 9/6574

(54) **HETEROZYKLISCHE AN EINER HYDROXYL-GRUPPE GESCHÜTZTE SELENA-MONOPHOSPHITE SOWIE VERFAHREN ZU DEREN HERSTELLUNG**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); WEILBEER, Claudia, 06406 Bernburg (DE); SELENT, Detlef, 18059 Rostock (DE); BÖRNER, Armin, 18059 Rostock (DE)

(57) **Zusammenfassung**

Neue heterozyklische an der Hydroxyl-Gruppe geschützte Selena-Monophosphite, Verfahren zu deren Herstellung als auch deren Verwendung als Ligand.

## Beschreibung

T. K. Paine beschreibt eine Synthese von 2,2'-Selenobis(4,6-di-tert-butylphenol) unter Verwendung von Selendioxid. Die Herstellung von 2,2'-Selenobis(4,6-di-tert-butylphenol) erfolgt hier in einem sauren Medium unter Zusatz von konzentrierter Salzsäure. Das Produkt wird mit einer Ausbeute von 25 % erhalten (T. K. Paine et al., "Manganese complexes of mixed O, X, O-donor ligands (X = S or Se): synthesis, characterization and catalytic reactivity", Dalton Trans., 2003, 15, 3136-3144). Hierbei ist besonders nachteilig, dass die Ausbeuten sehr gering und somit verbesserungswürdig sind.

Eine weitere mehrstufige Syntheseroute unter Verwendung von Grignard-Reagenz offenbart H. M. Lin et al., "A novel and efficient synthesis of selenides", ARKIVOC, 2012, viii, 146-156. Es wird eine Syntheseroute für Selenobiarylether offenbart in der zunächst Brom an das entsprechende Phenol addiert werden muss, um das Produkt dann mit Magnesium zu einem Grignard-Reagenz umzusetzen. Das Grignard-Reagenz kann dann mit dem zugesetzten Selen reagieren, bevor die eigentliche Kupplung zum Biarylether erfolgt:

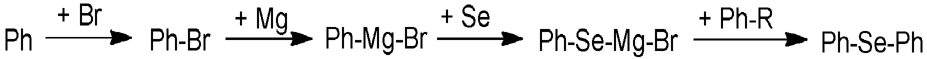

Das Produkt konnte in einer guten Ausbeute erzielt werden, jedoch ist diese Syntheseroute sehr aufwendig, was es für einen großtechnischen Einsatz unattraktiv macht. Hierbei sind eine Vielzahl von Syntheseschritten notwendig, die zum Teil nicht unkritisch in ihrer Durchführung sind, insbesondere wenn man an eine Hochskalierung denkt und Massstäbe verwendet, die in der Industrie üblich sind. Weiterhin fallen bei dieser Syntheseroute große Mengen an Abfallprodukten und Lösungsmittel an, die aufwändig entsorgt werden müssen, unter anderem auf Grund des Einsatzes von Brom.

Eine grosstechnisch wirtschaftliche Syntheseroute zur Herstellung von Selenodiphenolen beschreibt die EP 15168645.8 bzw. US 14/720,063.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxidierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Rhodium-Monophosphit-Komplexe in katalytisch aktiven Zusammensetzungen sind geeignet für die Hydroformylierung von verzweigten Olefinen mit innenständigen Doppelbindungen. Seit den 1970er Jahren ist die Verwendung von so genannten "bulky phosphites" in der Hydroformylierung beschrieben (siehe u.a. van Leeuwen et al., Journal of Catalysis, 2013, 298, 198-205). Diese zeichnen sich durch eine gute Aktivität aus, jedoch ist die n/i-Selektivität für endständig oxidierte Verbindungen gering und verbesserungswürdig.

In diesen Hydroformylierungen werden in der Regel Mono- und Bisphosphite eingesetzt, die oftmals aus Biphenol-Bausteinen aufgebaut sind. Die Entwicklung neuer Liganden ist häufig durch die zur Verfügung stehenden Biphenol-, also Ligandenbausteine limitiert. So stellen 2,2'-Selenobiarylether sowie Diphenylselenoxide und Diphenylselenide eine hochinteressante Verbindungsklasse dar. Die 2,2'-Selenobiarylether werden derzeit nur in bestimmten Komplexen, vor allem manganhaltigen, verwendet, sie besitzen aber ein großes Potential für weitere Anwendungen.

Aufgabe der Erfindung war es, eine weitere gänzlich neue Substanzklasse an Liganden herzustellen, um das Feld der verfügbaren Liganden für die jeweiligen spezifischen Komplexe in der Katalyse zu erweitern. Des Weiteren bestand die Aufgabe Liganden für Rhodium-Hydroformylierungskatalysatoren herzustellen.

Gelöst werden die Aufgaben mit den heterozyklischen Selena-Phosphiten nach Anspruch 1, dem Verfahren nach Anspruch 7 sowie der Verwendung nach Anspruch 15. Besondere Ausführungsformen sind in den Unteransprüchen sowie detailliert in der Beschreibung offenbart. Gelöst werden die Aufgaben ebenso bevorzugt mit Selena-Phosphiten der Strukturen **I** und **Ia**, insbesondere mit -R^{1*} ausgewählt aus Struktur **II**, **IV**, **V**, **VI** und **VII**. Dabei sind die Wasserstoff, Alkyl und/oder -O-(C₁-C₁₂)-Alkyl substituierten Verbindungen der Strukturen **II**, **IV**, **V**, **VI** und **VII** der organofunktionellen Phosphit-Gruppe -R^{1*}, in Kombination mit der Gruppe -R¹ die ausgewählt ist aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₆-C₂₀)-Aryl besonders bevorzugte Verbindungen. Gegenstand der Erfindung ist mindestens eine Verbindung von heterozyklische Selena-Phosphiten, welche eine allgemeine Struktur **I** aufweist 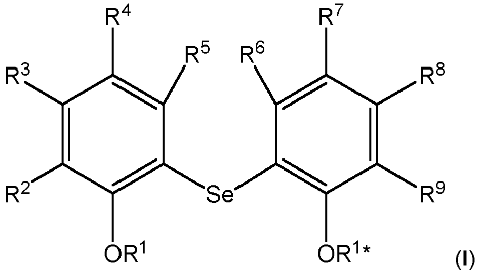 wobei R², R³, R⁴, R⁵, R⁶, R⁷, R⁸ und R⁹ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=0-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen linear, verzweigt oder cyclisch sein können, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe, mindestens einen Substituenten aufweisen kann, und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und wobei die Gruppe -R¹ ausgewählt sein kann aus: -(C₁-C₁₂)-Alkyl, -(C₁-C₁₂)-Alkyl-O-(C₁-C₁₂)-Alkyl, -(C₆-C₂₀)-Aryl, -(C₆-C₂₀)-Aryl -O-(C₁-C₁₂)Alkyl, -(C₁-C₁₂)-Alkyl-O-(C₆-C₂₀)-Aryl, -(C₆-C₂₀)-Aryl-O-(C₆-C₂₀)-Aryl, -(CO)O-(C₁-C₁₂)-Alkyl, -acetyl, wobei die Alkylgruppen linear, verzweigt oder cyclisch sein können, wobei die genannten Alkyl- und Arylgruppen jeweils unabhängig unsubstituiert oder substituiert sein können, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
und die Gruppe -R^{1*} einer organofunktionellen Phosphit-Gruppe entspricht.

Entsprechend einer besonders bevorzugten Alternative kann das heterozyklische Selena-Phosphit der allgemeinen Struktur **I** als -R^{1*} eine organofunktionelle Phosphit-Gruppe ausgewählt aus den Strukturen **II**, **III, IV**, **V**, **VI** und **VII** aufweisen, 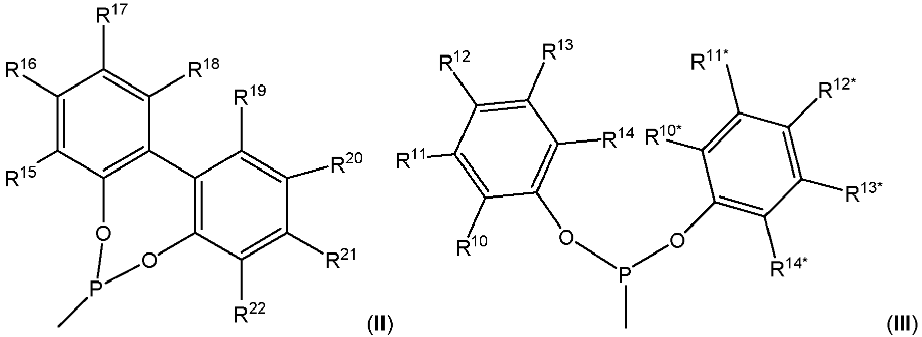 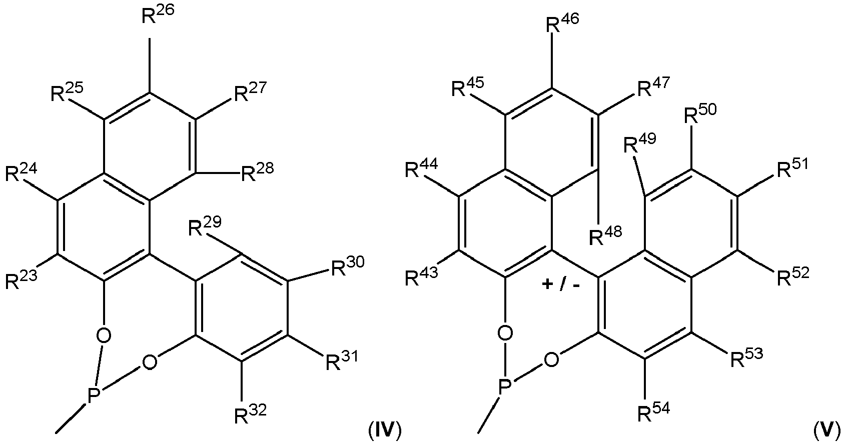 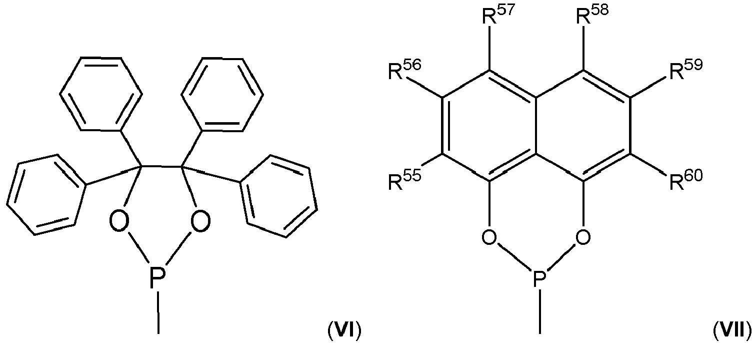 wobei die Reste R¹⁵, R¹⁶, R¹⁷, R¹⁸, R¹⁹, R²⁰, R²¹, R²² -in Struktur **II**, R¹⁰, R¹¹, R¹², R¹³, R¹⁴, R^{10*}, R^{11*}, R^{12*}, R^{13*}, R^{14*} in der Struktur **III**, R²³, R²⁴, R²⁵, R²⁶, R²⁷, R²⁸, R²⁹, R³⁰, R³¹ und R³² in Struktur **IV**, R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur **V**, R⁵⁵, R⁵⁶, R⁵⁷, R⁵⁸, R⁵⁹ und R⁶⁰ in Struktur **VII**, jeweils unabhängig in der jeweiligen Struktur voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sein können, wobei die Alkyl- und Arylgruppen jeweils unabhängig unsubstituiert oder substituiert sein können, wobei die jeweilige substituierte -(C₁-C₁₂)-Alkylgruppe und die jeweilige substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Gleichfalls Gegenstand der Erfindung sind Verbindungen des heterozyklischen Selena-Phosphits der allgemeinen Struktur **I** in der die Gruppe -R¹ ausgewählt sein kann aus: -(C₁-C₁₂)-Alkyl, -(C₁-C₁₂)-Alkyl-O-(C₁-C₁₂)-Alkyl, -(C₆-C₂₀)-Aryl, -(C₆-C₂₀)-Aryl-O-(C₁-C₁₂)Alkyl, -(C₁-C₁₂)-Alkyl-O-(C₆-C₂₀)-Aryl, -(C₆-C₂₀)-Aryl-O-(C₆-C₂₀)-Aryl, - (C=O)-O-(C₁-C₁₂)-Alkyl, wobei die Alkylgruppen linear, verzweigt oder cyclisch sein können, wobei die genannten Alkyl- und Arylgruppen jeweils unabhängig unsubstituiert oder substituiert sein können, wobei substituierte -(C₁-C₁₂)-Alkylgruppen und substituierte -(C₆-C₂₀)-Arylgruppen mindestens einen Substituenten aufweisen können und der mindestens eine Substituent jeweils unabhängig ausgewählt ist aus -(C₃-C₁₂)-Cycloalkyl und/oder -(C₆-C₂₀)-Aryl.

Nach einer bevorzugten Ausführungsvariante kann das heterozyklische Selena-Phosphit der allgemeinen Struktur **I** ausgewählt sein aus mindestens einer Verbindung der Struktur **Ia** 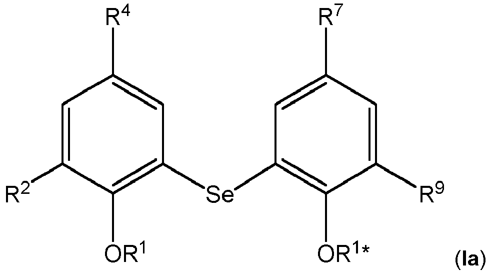 wobei R², R⁴, R⁷ und R⁹ jeweils unabhängig voneinander ausgewählt sein können aus -(C₁-C₁₂)-Alkyl, -O-(C₁-C₁₂)-Alkyl, -(C₆-C₂₀)-Aryl, -O-(C₆-C₂₀)-Aryl, -Halogen, und
wobei die Gruppe -R¹ ausgewählt sein kann aus -(C₁-C₁₂)-Alkyl, -(C₁-C₁₂)-Alkyl-O-(C₁-C₁₂)-Alkyl, -(C₆-C₂₀)-Aryl, -(C₆-C₂₀)-Aryl-O-(C₁-C₁₂)-Alkyl, -(C₁-C₁₂)-Alkyl-O-(C₆-C₂₀)-Aryl, -(C₆-C₂₀)-Aryl-O-(C₆-C₂₀)-Aryl, und die Gruppe -R^{1*} in Struktur **Ia** einer organofunktionellen Phosphit-Gruppe entsprechen kann, insbesondere einer der Phosphitgruppen der Strukturen **II**, **III**, **IV**, **V**, **VI** oder **VII**.

Entsprechend einer besonders bevorzugten Ausführungsform kann -R¹ jeweils unabhängig voneinander in den Strukturen **I** oder **Ia** ausgewählt sein aus Methoxymethyl- (-CH₂OCH₃ (MOM)), -CH₂OCH₂C₆H₅ (BOM), -CH₂OCH₂CH₂OCH₃ (MEM), Benzyl-, Methyl-, *tert*.-Butyl, iso-Pentyl.

Gleichfalls bevorzugte Verbindungen umfassen heterozyklische Selena-Phosphite der allgemeinen Struktur **I** oder **Ia** in denen -R^{1*} ausgewählt sein kann aus den Strukturen **II**, **III, IV**, **V**, **VI** und **VII** 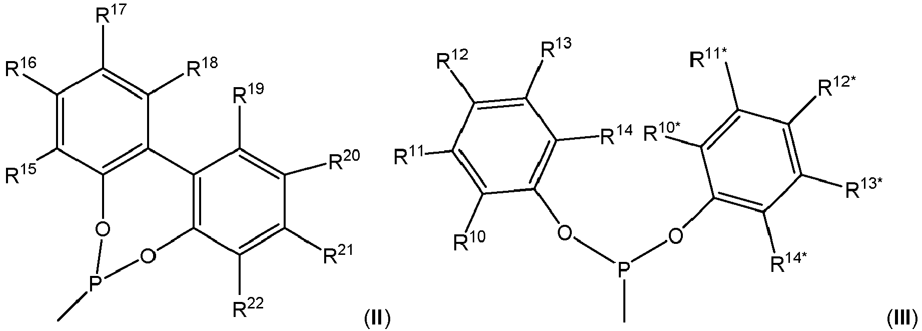 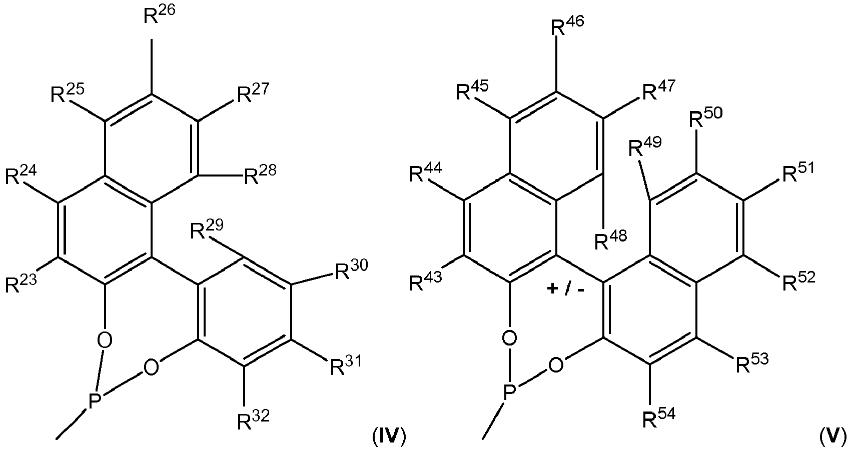 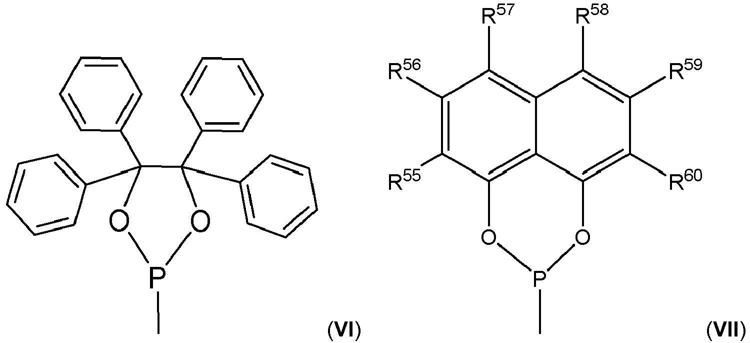 wobei die Reste R¹⁵ R¹⁶, R¹⁷, R¹⁸ R¹⁹, R²⁰, R²¹, R²² in Struktur **II**, R¹⁰, R¹¹, R¹², R¹³, R¹⁴, R^{10*}, R^{11*}, R^{12*}, R^{13*}, R^{14*} in der Struktur **III,** R²³, R²⁴, R²⁵, R²⁶, R²⁷, R²⁸, R²⁹, R³⁰, R³¹ und R³² in Struktur **IV**, R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur **V**, R⁵⁵, R⁵⁶, R⁵⁷, R⁵⁸, R⁵⁹ und R⁶⁰ in Struktur **VII**, jeweils für jede Struktur unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sind.

Nach einer weiteren bevorzugten Alternative kann in dem heterozyklischen Selena-Phosphit der allgemeinen Struktur **I** oder **Ia** -R^{1*} ausgewählt sein aus der Struktur **III,** und die Gruppe -R¹ kann ausgewählt sein aus -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₆-C₂₀)-Aryl, wobei die Alkylgruppen linear, verzweigt oder cyclisch sind, 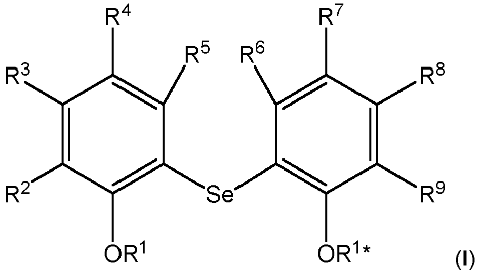 wobei R², R³, R⁴, R⁵, R⁶, R⁷, R⁸ und R⁹ in Struktur **I** jeweils unabhängig voneinander ausgewählt sein können aus: -H, -(C₁-C₁₂)-Alkyl, -O-(C₁-C₁₂)-Alkyl, wobei die Alkylgruppen linear, verzweigt oder cyclisch sind, oder 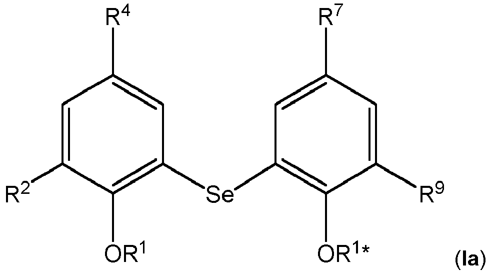 wobei R², R⁴, R⁷ und R⁹ in Struktur **Ia** jeweils unabhängig voneinander ausgewählt sein können aus: -(C₁-C₁₂)-Alkyl, -O-(C₁-C₁₂)-Alkyl, wobei die Alkylgruppen linear, verzweigt oder cyclisch sind, und 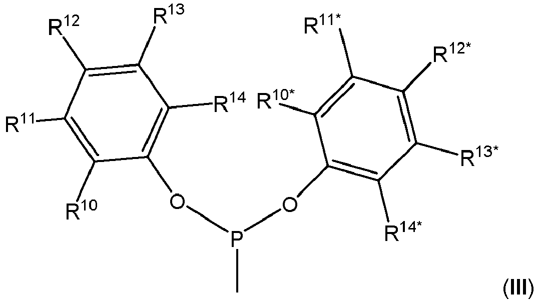 mit R¹⁰, R¹¹, R¹², R¹³, R¹⁴, R^{10*}, R^{11*}, R^{12*}, R^{13*}, R^{14*} in der Struktur **III,** jeweils unabhängig voneinander ausgewählt sein können aus: -H, -(C₁-C₁₂)-Alkyl, -O-(C₁-C₁₂)-Alkyl.

Entsprechend weiteren bevorzugten Alternativen können -R¹ und -R^{1*} im heterozyklischen Selena-Phosphit der allgemeinen Struktur **I** oder **Ia** ausgewählt sein aus den Alternativen
mit -R^{1*} ist ausgewählt aus **II**, **III, IV**, **V**, **VI** und **VII,** wobei die Reste in den Strukturen **II**, **III**, **IV**, **V**, **VI** und **VII** ausgewählt sind aus -H, -(C₁-C₁₂)-Alkyl, -O-(C₁-C₁₂)-Alkyl und
mit -R¹ ist ausgewählt aus Methoxymethyl- (-CH₂OCH₃ (MOM)), -CH₂OCH₂C₆H₅ (BOM), -CH₂OCH₂CH₂OCH₃ (MEM), Benzyl-, Methyl-, *tert*.-Butyl, iso-Pentyl.

In Struktur **Ia** können R², R⁴, R⁷ und R⁹ jeweils unabhängig voneinander ausgewählt sein aus: Methyl-, Ethyl-, *tert*-Butyl-, iso-Pentyl-, Methoxy-, Benzyl-, -Halogen.

In der Struktur **II** können R¹⁵, R¹⁶, R¹⁷, R¹⁸, R¹⁹, R²⁰, R²¹, R²² jeweils unabhängig ausgewählt sein aus: -H, Methyl-, Ethyl-, *tert*-Butyl-, iso-Pentyl-, Methoxy-, Benzyl-, Halogen.

In der Struktur **III** können R¹⁰, R¹¹, R¹², R¹³, R¹⁴, R^{10*}, R^{11*}, R^{12*}, R^{13*}, R^{14*} jeweils unabhängig ausgewählt sein aus: -H, Methyl-, Ethyl-, *tert*-Butyl-, iso-Pentyl-, Methoxy-, Benzyl-, Halogen-.

In der Struktur **VIII** können R⁵⁵, R⁵⁶, R⁵⁷, R⁵⁸, R⁵⁹ und R⁶⁰ jeweils unabhängig ausgewählt sein aus: -H, Methyl-, Ethyl-, *tert*-Butyl-, iso-Pentyl-, Methoxy-, Benzyl-, Halogen-.

In Struktur **Ia** können R², R⁴, R⁷ und R⁹ jeweils unabhängig voneinander ausgewählt sein aus: Methyl-, Ethyl-, *tert*-Butyl-, iso-Pentyl-, Methoxy-, Benzyl-, -Halogen, wobei -R¹ ausgewählt sein kann aus Methoxymethyl- (-CH₂OCH₃ (MOM)), -CH₂OCH₂C₆H₅ (BOM), -CH₂OCH₂CH₂OCH₃ (MEM), Benzyl-, Methyl-, *tert*.-Butyl, iso-Pentyl und -R^{1*} kann ausgewählt sein aus Struktur **III** wobei R¹⁰, R¹¹, R¹², R¹³, R¹⁴, R^{10*}, R^{11*}, R^{12*}, R1^{3*}, R^{14*} jeweils unabhängig ausgewählt sein können aus: -H, Methyl-, Ethyl-, *tert*-Butyl-, iso-Pentyl-, Methoxy-, Benzyl-, Halogen-. Bevorzugt sind R¹¹, R¹³, R¹⁴, R^{11*}, R^{13*}, R^{14*} -H und R¹⁰, R¹², R^{10*}, R^{12*} ausgewählt aus Methyl-, Ethyl-, *tert*-Butyl-, iso-Pentyl-.

Gleichfalls Gegenstand der Erfindung ist ein Verfahren zur Herstellung mindestens eines heterozyklischen Selena-Phosphites der allgemeinen Struktur **I** 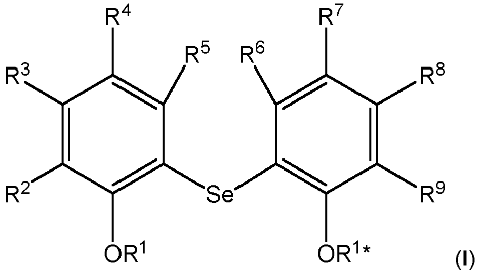 wobei R², R³, R⁴, R⁵, R⁶, R⁷, R⁸ und R⁹ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=0-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen linear, verzweigt oder cyclisch sein können, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe, mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei die Gruppe -R¹ ausgewählt sein kann aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₁-C₁₂)Alkyl, -(C₆-C₂₀)-Aryl-O-(C₆-C₂₀)-Aryl, -(C=O)-O-(C₁-C₁₂)-Alkyl, -acetyl, wobei die Alkylgruppe linear, verzweigt oder cyclisch sein kann, wobei die genannten Alkyl- und Arylgruppen jeweils unabhängig unsubstituiert oder substituiert sein können, wobei substituierte -(C₁-C₁₂)-Alkylgruppen und substituierte -(C₆-C₂₀)-Arylgruppen mindestens einen Substituenten aufweisen können und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
und die Gruppe -R^{1*} einer organofunktionellen Phosphit-Gruppe entspricht.
umfassend mindestens den Verfahrensschritt
(i) Umsetzen eines Selenodiaryls der allgemeinen Struktur **IX** 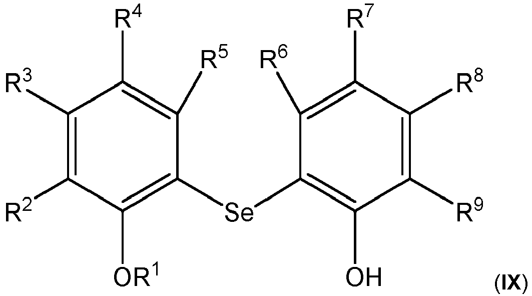 wobei R², R³, R⁴, R⁵, R⁶, R⁷, R⁸ und R⁹ jeweils unabhängig voneinander ausgewählt sein können aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=0-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen linear, verzweigt oder cyclisch sein können, wobei die Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe, mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
   und Gruppe -R¹ ausgewählt sein kann aus: -(C₁-C₁₂)-Alkyl, -(C₁-C₁₂)-Alkyl-O-(C₁-C₁₂)-Alkyl, -(C₆-C₂₀)-Aryl, -(C₆-C₂₀)-Aryl-O-(C₁-C₁₂)Alkyl, -(C₁-C₁₂)-Alkyl-O-(C₆-C₂₀)-Aryl, -(C₆-C₂₀)-Aryl-O-(C₆-C₂₀)-Aryl, -(C=O)-O-(C₁-C₁₂)-Alkyl, -acetyl, wobei die Alkylgruppe linear, verzweigt oder cyclisch sein kann, wobei die genannten Alkyl- und Arylgruppen jeweils unabhängig unsubstituiert oder substituiert sein können, wobei die jeweilige substituierte -(C₁-C₁₂)-Alkylgruppe und substituierte -(C₆-C₂₀)-Arylgruppe mindestens einen Substituenten aufweisen kann und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
(ii) mit mindestens einer Halogenphosphit-Verbindung R^{1*}Hal der Formel **X**, wobei Hai jeweils unabhängig ausgewählt ist aus Fluor, Chlor, Brom, Jod, bevorzugt ist Hai gleich Chlor, und wobei
   -R^{1*} einer organofunktionellen Phosphit-Gruppe entspricht,
(iii) und erhalten mindestens eines heterozyklischen Selena-Phosphits der allgemeinen Struktur **I**.

Entsprechend einer bevorzugten Ausführungsform wird in dem Verfahren vorzugsweise als Halogenphosphit-Verbindung R^{1*}Hal der Formel **X** mit Hal jeweils unabhängig ausgewählt aus Fluor, Chlor, Brom, Jod eingesetzt, bevorzugt ist Hal gleich Chlor, und -R^{1*} kann jeweils ausgewählt sein aus den Strukturen **II**, **III**, **IV**, **V**, **VI** und **VII**, 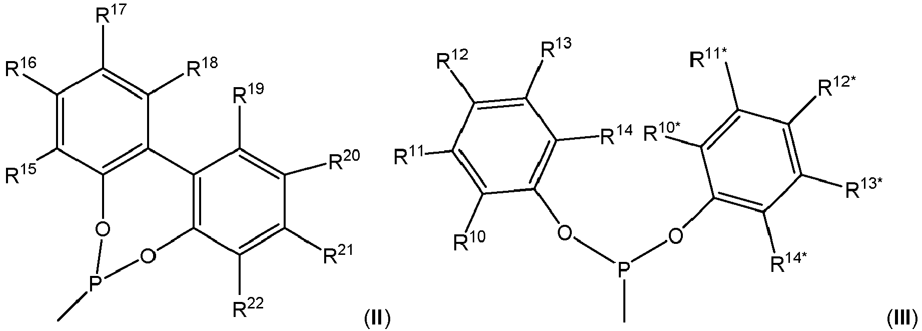 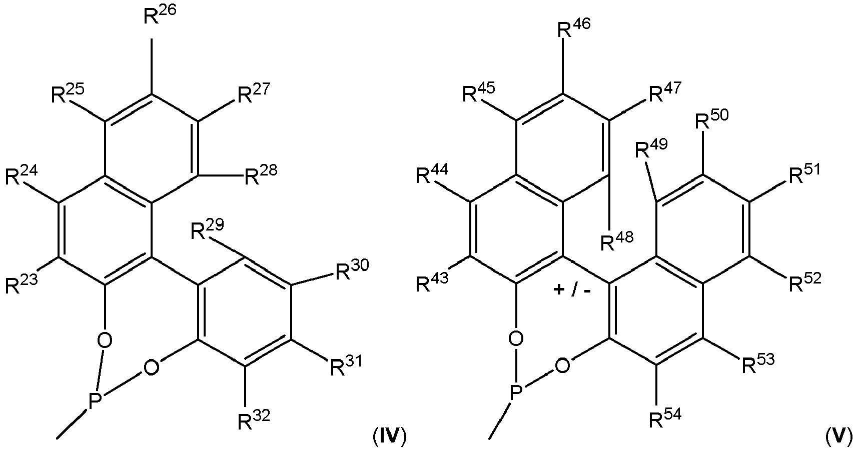 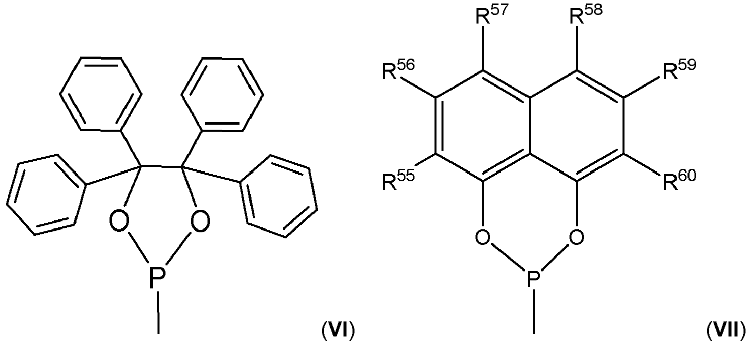 wobei die Reste R¹⁵ R¹⁶, R¹⁷, R¹⁸ R¹⁹, R²⁰, R²¹, R²² in Struktur **II**, R¹⁰, R¹¹, R¹², R¹³, R¹⁴, R^{10*}, R^{11*}, R^{12*}, R^{13*}, R^{14*} in der Struktur **III,** R²³, R²⁴, R²⁵, R²⁶, R²⁷, R²⁸, R²⁹, R³⁰, R³¹ und R³² in Struktur **IV**, R⁴³, R⁴⁴, R⁴⁵, R⁴⁶, R⁴⁷ R⁴⁸, R⁴⁹, R⁵⁰, R⁵¹, R⁵², R⁵³ und R⁵⁴ in Struktur **V**, R⁵⁵, R⁵⁶, R⁵⁷, R⁵⁸, R⁵⁹ und R⁶⁰ in Struktur **VII**, jeweils in jeder Struktur unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sein können, wobei die Alkyl- und Arylgruppen jeweils unabhängig unsubstituiert oder substituiert sein können, wobei die jeweilige substituierte -(C₁-C₁₂)-Alkylgruppe und die jeweilige substituierte -(C₆-C₂₀)-Arylgruppe mindestens einen Substituenten aufweisen können und der mindestens eine Substituent jeweils unabhängig ausgewählt sein kann aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Ferner ist es besonders bevorzugt, wenn in dem Verfahren eine Halogenphosphit-Verbindung R^{1*}Hal der Formel **X** mit Hal ausgewählt aus Chlor, und -R^{1*} ausgewählt aus den Strukturen **II**, **III, IV**, **V**, **VI** und **VII,** eingesetzt wird, dabei können die Reste R¹⁵, R¹⁶, R¹⁷, R¹⁸, R¹⁹, R²⁰, R²¹, R²² in Struktur **II,** R¹⁰, R¹¹, R¹², R¹³, R¹⁴, R^{10*}, R^{11*}, R^{12*}, R^{13*}, R^{14*} in der Struktur **III,** R²³, R²⁴, R²⁵, R²⁶, R²⁷, R²⁸, R²⁹, R³⁰, R³¹ und R³² in Struktur **IV**, R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur **V**, R⁵⁵, R⁵⁶, R⁵⁷, R⁵⁸, R⁵⁹ und R⁶⁰ in Struktur **VII**, jeweils unabhängig voneinander ausgewählt sein aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sind.

Nach einer besonders bevorzugten Verfahrensalternative kann als Selenodiaryl ein Selenodiaryl der allgemeinen Struktur **IXa** eingesetzt werden 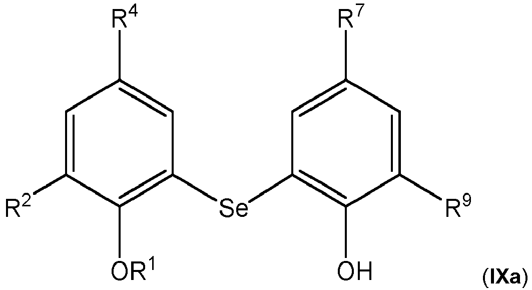 wobei R², R⁴, R⁷ und R⁹ jeweils unabhängig voneinander ausgewählt sein können aus: -(C₁-C₁₂)-Alkyl, -O-(C₁-C₁₂)-Alkyl, -(C₆-C₂₀)-Aryl, -O-(C₆-C₂₀)-Aryl, -Halogen, und wobei die Gruppe -R¹ ausgewählt sein kann aus: -(C₁-C₁₂)-Alkyl, -(C₁-C₁₂)-Alkyl-O-(C₁-C₁₂)-Alkyl, -(C₆-C₂₀)-Aryl, -(C₆-C₂₀)-Aryl-O-(C₁-C₁₂)Alkyl, -(C₁-C₁₂)-Alkyl-O-(C₆-C₂₀)-Aryl, -(C₆-C₂₀)-Aryl-O-(C₆-C₂₀)-Aryl, wobei die Alkylgruppen linear, verzweigt oder cyclisch sind.

Die Umsetzung der Verbindung der Struktur **I** kann dabei erfolgen, in dem die Halogenphosphit-Verbindung R^{1*}Hal in einem Lösemittel vorgelegt wird und das Selenodiaryl der Struktur **I** oder **Ia** optional im Gemisch mit einer Base, vorzugsweise einem Amin, bevorzugt einem Alkylamin, und optional in einem Lösemittel zugesetzt wird. Alternativ kann die Umsetzung erfolgen indem das Selenodiaryl in einem Lösemittel vorgelegt wird und die Halogenphosphit-Verbindung optional in einem Lösemittel zugesetzt wird.

Gleichfalls Gegenstand der Erfindung ist ein Verfahren in dem (i) die Umsetzung in Gegenwart einer Base erfolgt, insbesondere eines Amins oder einer Pyridinbase, vorzugsweise eines Alkylamins, wie Triethylamin oder Dimethylaminobutan, besonders bevorzugt Triethylamin.

Ferner ist Gegenstand der Erfindung ein Verfahren in dem das Selenodiaryl der allgemeinen Struktur **IX** oder **IXa** mit R^{1*}Hal der Formel **X** im molaren Verhältnis von 10 : 1 bis 1 : 10 umgesetzt wird, vorzugsweise im Verhältnis von 4: 1 bis 1 : 4, besonders bevorzugt im Verhältnis von 2,5 : 1 bis 1 : 2,5. Bevorzugt ist Hal Chlor oder Brom.

Des Weiteren erfolgt die (i) Umsetzung vorzugsweise bei einer Temperatur von -45 °C bis 80 °C, besonders von -15 bis 30°C, besonders bevorzugt von -5 bis 5°C.

Ferner erfolgt (i) die Umsetzung vorzugsweise in einem aprotischen Lösemittel, insbesondere ist das Lösemittel ausgewählt aus organischen aromatischen, halogenierten Lösemitteln oder Kohlenwasserstoffen.

Nach einer weiteren Ausführungsvariante ist Gegenstand der Erfindung die Verwendung eines heterozyklischen Selena-Phosphits der Struktur **I** oder **Ia** als Ligand.

Der Begriff Phenol, Aryl, Phosphit wird in dieser Anmeldung als Gattungsbegriff verwendet und umfasst somit auch substituierte Strukturen der genannten Verbindungen.

Die vorgenannten Definitionen für substituierte -(C₁-C₁₂)-Alkylgruppen und substituierte -(C₆-C₂₀)-Arylgruppen gelten für alle Gruppen in denen diese Alkyl- oder Arylgruppen enthalten sind, also insbesondere auch für die folgenden Gruppen: -O-(C₁-C₁₂)-Alkyl, -(C₆-C₂₀)-Aryl, -O-(C₆-C₂₀)-Aryl, -OC=0-(C₁-C₁₂)-Alkyl, -(C=O)-O-(C₁-C₁₂)-Alkyl.

Ein oder mehrere Substituenten in den vorgenannten Strukturen der Selena-Phosphite und Selenodiarylen umfassen vorzugsweise 1 bis 10 Substituenten, insbesondere 1 bis 3.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl-, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Halogen als Substituent an Alkyl oder Aryl umfasst Fluor, Chlor, Brom und Jod, wobei Chlor und Fluor besonders bevorzugt sind.

Alle Erläuterungen zum Ausdruck -(C₁-C₁₂)-Alkyl in den vorgenannten erfindungsgemäßen Strukturen der Selena-Phosphite und Selenodiarylen gelten auch für die Alkylgruppen in -O-(C₁-C₁₂)-Alkyl, also in -(C₁-C₁₂)-Alkoxy.

Bevorzugt sind unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen in den vorgenannten Strukturen der Selena-Phosphite und Selenodiarylen können in Abhängigkeit von ihrer Kettenlänge, einen oder mehrere Substituenten aufweisen. Die Substituenten sind vorzugsweise unabhängig voneinander ausgewählt aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Diese Definition gilt für alle substituierten Alkyl- oder Alkyoxygruppen der vorliegenden Erfindung.

Alle Erläuterungen zum Ausdruck -(C₆-C₂₀)-Aryl in den vorgenannten erfindungsgemäßen Strukturen der Selena-Phosphite und Selenodiarylen gelten auch für die Arylgruppen in -O-(C₆-C₂₀)-Aryl.

Bevorzugt sind unsubstituierte -O-(C₆-C₂₀)-Gruppen.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl. Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltigen Gruppen sind vorzugsweise ausgewählt aus -O-, -S-, -N-, -N(=O)-, -C(=O)- oder -S(=O)-. Beispiele für -(C₃-C₁₂)-Heterocycloalkylgruppen sind Tetrahydrothiophenyl, Tetrahydrofuryl, Tetrahydropyranyl und Dioxanyl.

Nachfolgend wird die Erfindung näher an Beispielen erläutert, ohne die Erfindung auf die Ausführungsbeispiele zu beschränken.

### Allgemeine Methoden

### Lösungsmittel und Reagenzien

Alle Reaktionen mit feuchtigkeits- und/oder sauerstoffempfindlichen Substanzen wurden in ausgeheizten Apparaturen unter Argonatmosphäre durchgeführt. Lösungsmittel zur Extraktion und Säulenchromatographie wurden in folgenden Reinheiten verwendet: Dichlormethan (99.9%, Fa. *Walter*, Art.-Nr. BIE 073107033) Ethylacetat (99.5%, Fa. *Walter*, Art.-Nr. BIE 003917025) und *n-*Hexan (95%, Fa. *Walter (Baker),* Art.-Nr. 8669),
*n*-Heptan (95%, Fa. *Walter (Baker),* Art.-Nr. 8662). Andere Lösungsmittel für Extraktion und Säulenchromatographie waren von technischer Qualität und wurden, sofern nicht anders vermerkt, ohne weitere Reinigung eingesetzt. Trockene Lösungsmittel (abs.) wurden mit einem Pure Solv MD-7 System gereinigt und unter Argonatmosphäre aufbewahrt. Benzylbromid wurde vor dem Gebrauch frisch destilliert (17 mbar/82°C). Deuterierte Lösungsmittel wurden von den angegebenen Trockenmitteln destilliert: Dichlormethan-d₂ (Phosphorpentoxid), Toluol-d₈ (1. KOH; 2. Natrium). Für die Synthesen wurden Chemikalien der Firmen *Sigma Aldrich, Alfa Aesar, Acros Organics, Avantor Performance Materials B.V., Merck KGaA* und *ABCR GmbH* & *Co. KG* verwendet. Diese wurden, sofern nicht anders vermerkt, ohne weitere Reinigung eingesetzt.

Filtration: Filtrationen zur Abtrennung von entstandenen Feststoffen wurden mit einer G4-Fritte (Porenweite: 10-16 µm) durchgeführt.

### Analytik

IR-Spektroskopie: Die IR-Spektren wurden mit dem FT-IR-Spektrometer *Nicolet 6700* der Firma *Thermo Electron* aufgenommen. Die Substanzen wurden mittels ATR-Verfahren vermessen.

¹H-NMR-Spektroskopie: Die ¹H-NMR-Spektren wurden mit dem Modell *AV 300* (300 MHz) sowie mit dem Modell *Fourier 300* (300 MHz) der Firma *Bruker* aufgenommen. Die chemischen Verschiebungen sind in Einheiten der δ-Skala angegeben. Die Restprotonensignale der Lösungsmittel (Dichlormethan-d₂: δ = 5.32 ppm, Toluol-d₈: δ = 7.09; 7.00; 6.98; 2.09 ppm) dienten dabei als Standard.

¹³C-NMR-Spektroskopie: Die ¹³C-NMR-Spektren wurden mit den Modellen *AV 300* (75 MHz) und *Fourier 300* (75 MHz) der Firma *Bruker* aufgenommen. Als interner Standard diente das Signal des Lösungsmittels (Dichlormethan-d₂: δ = 54.0 ppm, Toluol-d₈: δ = 137.9; 129.2; 128.3; 125.5; 20.4 ppm) wobei die chemischen Verschiebungen den ¹H-breitbandentkoppelten Spektren entnommen wurden.

⁷⁷Se-NMR-Spektroskopie: Die ⁷⁷Se-NMR-Spektren wurden mit dem *AV 300* (57 MHz) der Firma *Bruker* aufgenommen. Die Spektren wurden ¹H-breitbandentkoppelt vermessen. Die chemischen Verschiebungen sind in ppm angegeben.

Massenspektrometrie: EI-Massespektren wurden am Gerät *Finnigan MAT 95-XP* der Firma *Thermo Electron* sowie ESI-TOF-Massespektren mit dem Modell *6210 Time-of-Flight LC*/*MS* der Firma *Agilent* aufgenommen.

### Autoklaven-Versuche der Rhodium-katalysierten Hydroformylierung

Die Hydroformylierung wurde in einem mit Druckkonstanthaltung, Gasflussmessung, Begasungsrührer und Druckpipette ausgestatteten 200 mL-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat 1-Octen oder n-Octene (EVONIK Industries AG, Octenisomerengemisch aus 1-Octen: 3,3 %; cis+trans-2-Octen: 48.5%; cis+trans-3-Octen: 29.2%; cis+trans-Octen-4: 16.4%; gerüstisomere Octene: 2.6%) wurde mehrere Stunden über Natrium am Rückfluss erhitzt und unter Argon destilliert.

Für die Versuche wurden im Autoklaven unter Argonatmosphäre Lösungen der Katalysatorvorstufe und des Liganden gemischt. Als Katalysatorvorstufe kam [(acac)Rh(COD)] (*Umicore,* acac=Acetylacetonat-Anion; COD=1,5-Cyclooctadien) zum Einsatz. Für Versuche mit einer Konzentration von 100 ppm-m Rhodium wurden 10 mL einer 4.31 mM Lösung in den Autoklaven gegeben. Anschließend wurde die einem Verhältnis L/Rh = 5:1 (bzw. 1:1) entsprechende Masse des Liganden in 10 mL Toluol gelöst und zugemischt. Durch Zugabe von weiterem Toluol wurde das Anfangsvolumen der Katalysatorlösung auf 41.0 ml eingestellt. In eine druckfeste Pipette wurde eingefüllt: 1-Octen bzw. n-Octene (10.70 g). Der Autoklav wurde bei einem Gesamtgasdruck (Synthesegas: *Linde*; H₂(99.999%): CO(99.997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48.5 bar für einen Enddruck von 50 bar bzw. b) 19.5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. *Bronkhorst*, NL) über 4h geführt. Der Autoklav wurde nach Ablauf der Reaktionszeit auf Zimmertemperatur abgekühlt, unter Rühren entspannt und mit Argon gespült. Jeweils 1.0 mL der Reaktionsmischungen wurden unmittelbar nach Abschalten des Rührers entnommen, mit 5.0 mL Pentan verdünnt und gaschromatographisch analysiert: HP 5890 Series II plus, PONA, 50 m x 0.2 mm x 0.5 µm.

Einige Abkürzungen: Bn = Benzyl; ber. = berechnet; gef. = gefunden; MOM = Methylmethoxy;

### Synthese der Vorstufen:

### Allgemeine Arbeitsvorschrift - Diphenolselenide

8.2 mmol des jeweiligen Phenols werden im entsprechenden Lösungsmittel gelöst (8.2 m). Das Reaktionsgemisch wird erhitzt und 4.9 mmol Selendioxid werden unter Rühren zugegeben. Das Lösungsmittel wird im Vakuum destilliert (Temperatur <70 °C). Eine Fritte wird mit 2,5 cm Kieselgel (unten) und 2,5 cm Zeolith (oben) vorbereitet. Der Destillationsrückstand wird im Laufmittel aufgenommen und auf die Filtrationssäule geben. Mit Cyclohexan:Essigester (95:5) wird das Produkt von der Fritte waschen und in Fraktionen gesammelt. Die Fraktionen die das Produkt enthalten werden zusammengefasst und destillativ vom Laufmittel befreit. Die erhaltenen Fraktionen werden aus Cyclohexan:Essigester 95:5 umkristallisiert. Dazu wird der feste Rückstand bei 50 °C gelöst und unlösliche Rückstände über eine Glasfritte abfiltriert. Aus der gesättigten Lösung kristallisiert das Reaktionsprodukt bei Raumtemperatur über Nacht. Die erhaltenen Kristalle werden nochmal mit kaltem Cyclohexan gewaschen.

Die Strukturformel zeigt jeweils das bei der Reaktion angefallene Hauptprodukt.

### Bis(3,5-dimethyl-2-hydroxyphenyl)selen; 1a

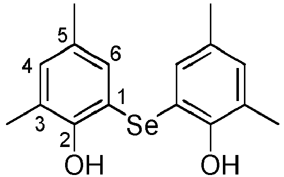

Die Durchführung der Reaktion erfolgt gemäß der allgemeinen Arbeitsvorschrift in einem verschraubbaren Reagenzglas. Hierzu werden 1.00 g (8.2 mmol, 1.0 Äquiv.) 2,4-Dimethylphenol und 0.54 g (4.9 mmol, 0.6 Äquiv.) Selendioxid in 1 mL Pyridin gelöst und erhitzt. Das Produkt wird als farbloser kristalliner Feststoff erhalten.

¹H-NMR (400 MHz, CDCl₃): δ (ppm) = 7.12 (s,2H, 6-H), 6.91 (s, 2H, 4-H), 5.97 (s,2H, OH), 2.23 (s, 6H, 3-CH₃) 2.23 (s, 6H, 5-CH₃); ¹³C-NMR (100 MHz), CDCl₃): δ (ppm) =151.7 (C-2),133.2 (C-3), 133.1 (C-5), 130.4 (C-4), 124.2 (C-6), 114.9 (C-1), 20.3 (5-CH₃), 16.5 (3-CH₃); ⁷⁷Se-NMR (76 MHz, CDCl₃): δ (ppm) = 163.36 ppm.

### Synthese der Chlorophosphite

Die Synthese der Monochlorophosphite wie 6-Chlorodibenzo[d,f][1,3,2]dioxaphosphepin ist dem Fachmann bekannt und erfolgt nach bekannter Weise. Chlorophosphite lassen sich durch Zusatz von Phosphortrichlorit in Gegenwart einer Base aus den entsprechenden Dihydroxyverbindungen herstellen. Für nähere Informationen siehe auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Synthese von Bis(2,4-di-tert-butylphenyl)phosphorchloridit X, 2a

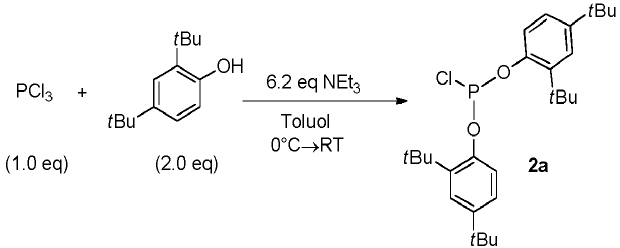

In einem ausgeheizten, unter Argon-Atmosphäre befindlichen 50 mL Schlenkgefäß wurden 412 mg (2.00 mmol, 2.0 eq) 2,4-Di-*tert*-Butylphenol und 873 µL (638 mg, 6.30 mmol, 6.3 eq) Triethylamin in 10 mL abs. Toluol vorgelegt und auf 0°C gekühlt. Anschließend wurden 87.5 µL (137 mg, 1.0 mmol, 1.0 eq) Phosphortrichlorid, gelöst in 2.0 mL abs. Toluol, zur gekühlten Lösung zugetropft, wobei ein farbloser Niederschlag gebildet wurde. Es wurde mit 5.0 mL abs. Toluol nachgespült und 24 h bei RT gerührt. Anschließend wurde das Reaktionsgemisch bis zum vollständigen Abtrennen des entstandenen Niederschlages filtriert und der Feststoff mit 10 mL abs. Toluol gewaschen. Das Lösungsmittel wurde unter vermindertem Druck entfernt und das Rohprodukt drei Stunden im Vakuum bei 50°C getrocknet. Es wurden 458 mg (0.962 mmol, 96%, 86%ig) der Titelverbindung 2a als farbloser Feststoff erhalten.

IR (ATR): *v̂* (cm⁻¹) = 2957; 2906; 2869; 1492; 1398; 1362; 1302; 1275; 1247; 1209; 1187; 1157; 1125; 1080; 1020; 941; 907; 887; 863; 819; 776; 745; 699; 645; 579; 532; 490; 464. ¹H-NMR (300 MHz, Toluol-d8): δ (ppm) = 7.54-7.40 (m, 4H, Ar-CH); 7.02 (dd, *J* = 8.5 Hz, *J* = 2.6 Hz, 2H, Ar-CH); 1.50 (s, 18H, -C(CH₃)₃); 1.24 (d, *J* = 0.9 Hz, 18H, -C(CH₃)₃);

¹³C-NMR (75 MHz, Toluol-d₈): δ (ppm)= 149.4; 147.1; 139.8 (d, *J* = 2.9 Hz); 124.8, 124.3; 119.9 (d, *J* = 16.2 Hz), 35.16; 34.61; 31.50; 30.41; ³¹P-NMR (122 MHz, Toluol-d₈): δ (ppm) = 161.6 ppm; MS (EI): m/z (%) = 476 (16.1) [C₂₈H₄₂ClO₂P]; 461 (100) [C₂₇H₃₉ClO₂P]⁻; 441 (1.69) [C₂₈H₄₂O₂P]⁻; HR-MS (EI): ber. für C₂₈H₄₂ClO₂P: 476.25760, gef.: 476.26031; ber. für C₂₈H₄₂³⁷ClO₂P: 478.25760, gef.: 478.25834; C₂₈H₄₂ClO₂P (476.26 g/mol).

### Synthese von 6-Chlorodibenzo[d,f][1,3,2]dioxaphosphepin X,

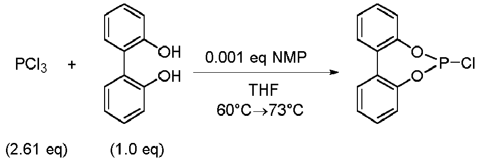

In einem ausgeheizten, unter Argon-Atmosphäre befindlichen 100 mL Dreihalskolben mit Rückflusskühler, Innenthermometer und Tropftrichter wurden 913 µL (1.43 g, 10.4 mmol, 2.61 eq) Phosphortrichlorid bei RT vorlegt und 3.80 µL *N*-Methyl-2-pyrrolidinon addiert. Das Reaktionsgemisch wurde auf 60°C Innentemperatur erhitzt. In einem separatem 10 mL Schlenkgefäß wurden 745 mg (4.00 mmol, 1.0 eq) 2,2-Biphenol in 20 mL abs. THF gelöst und innerhalb von 15 Minuten zur siedenden Reaktionslösung addiert. Es wurde mit 2.0 mL abs. THF nachgespült und auf eine Innentemperatur von 73°C erhitzt. Nach einer Reaktionszeit von 90 Minuten wurde die hellbraune Lösung auf RT abgekühlt und das Lösungsmittel unter verminderten Druck entfernt. Die braune Flüssigkeit wurde mit 10 mL abs. THF versetzt und zur Abtrennung des entstandenen Niederschlages filtriert. Das Lösungsmittel wurde erneut unter vermindertem Druck entfernt und das Rohprodukt drei Stunden im Vakuum bei 50°C getrocknet. Es wurden 962 mg (3.85 mmol, 96%, 99%ig) der Titelverbindung **X** als dunkelbraune Flüssigkeit erhalten.

**IR** (ATR): *v̂* (cm⁻¹) = 3064; 3027; 2924; 2848; 2435; 1919; 1601; 1565; 1498; 1474; 1432; 1295; 1273; 1244; 1194; 1172; 1116; 1094; 1042; 1010; 942; 904; 855; 760; 738; 708; 614; 597; 579; 530; 514; 486; 470; 451; 428; **¹H-NMR** (300 MHz, Toluol-d8): δ (ppm) = 7.29-6.83 (m, 8H, Ar-CH); **¹³C**-**NMR** (75 MHz, Toluol-d₈): δ (ppm) = 149.3 (d, *J* = 5.7 Hz); 137.1; 130.9 (d, *J* = 3.5 Hz); 130.0 (d, *J* = 1.6 Hz); 129.3; 126.0 (d, *J* = 1.2 Hz); 122.0 (d, *J* = 2.2 Hz); **³¹P-NMR** (122 MHz, Toluol-d₈): δ (ppm) = 180.5; **C₁₂H₈ClO₂P** (250.00 g/mol).

Entsprechend obiger Versuchsvorschriften können die folgende Monochlorophosphite der Struktur **X** gemäss Tabelle 1 hergestellt werden.

**Tabelle 1: Monochlorophosphite X**

| | | |
|---|---|---|
| 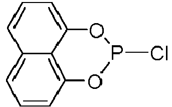 | 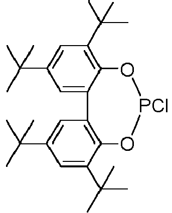 | 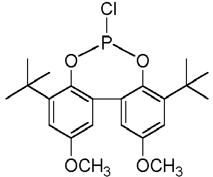 |
| Chloronaphtho[1,8-de][1,3,2]dioxaphosphinin | 2,2'-Bis-(3,5-ditert.-butyl)-phenol-chlorophosphit | 4,8-Di-tert-butyl-6-chloro-2,10-dimethoxydibenzo[d,f][1,3,2]dio xaphosphepin |
| 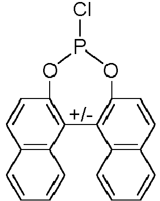 | 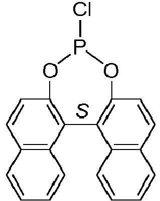 | 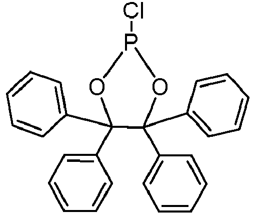 |
| rac-4-Chlor-dinaphtho[2,1-d:1',2'-f][1,3,2]dioxaphosphepin | 4-Chlor-S-dinaphtho[2,1-d:1',2'-f][1,3,2]dioxaphosphepin | 2-Chlor-4,4,5,5-tetraphenyl-1,3,2-dioxaphospholan |

### Synthese von 2-((2-(Benzyloxy)-3,5-dimethylphenyl)selanyl)-4,6-dimethylphenol IXa, 3a^{]}

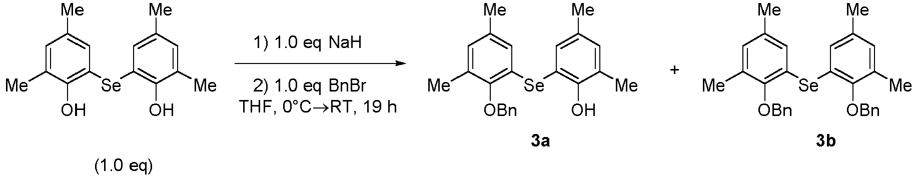

In einem ausgeheizten, unter Argon-Atmosphäre befindlichen 25 mL Schlenkgefäß wurden 40.2 mg (1.01 mmol, 1.0 eq, 60%ig in Paraffinöl) Natriumhydrid in 3.0 mL abs. THF suspendiert und auf 0°C gekühlt. Anschließend wurden 324 mg (1.01 mmol, 1.0 eq) Selenodiphenol, gelöst in 2.0 mL abs. THF, tropfenweise addiert. Die gelbliche Lösung wurde 15 Minuten bei 0°C und zwei Stunden bei RT gerührt. Anschließend wurden bei 0°C, 119 µL (172 mg, 1.01 mmol, 1.0 eq) Benzylbromid addiert und 30 Minuten bei 0°C gerührt. Nach weiteren 16 Stunden bei RT wurde das Lösungsmittel unter vermindertem Druck entfernt. Es wurden 391 mg des Reaktionsgemisches aus 2-((2-(Benzyloxy)-3,5-dimethylphenyl)selanyl)-4,6-dimethylphenol **3a** (314 mg, .762 mmol, 76%) und Bis(2-(benzyloxy)-3,5-dimethylphenyl)selan **3b** (Bn: Benzyl) (76.6 mg, 0.152 mmol, 15%) in einem Verhältnis von 4.96:1 (bestimmt aus Roh-¹H-NMR-Spektrum) erhalten. Das Reaktionsgemisch konnte durch säulenchromatographische Aufreinigung (100% H → 100:1 → 50:1 → 20:1 → 10:1 H/DCM) nicht vollständig aufgetrennt werden. 51.0 mg von **3a** konnten rein dargestellt werden (nachstehende analytische Auswertung).

**IR (ATR):** *v̂* (cm⁻¹) = 3409; 3030; 3011; 2919; 2854; 2730; 1567; 1497; 1467; 1372; 1328; 1284; 1268; 1251; 1231; 1208; 1123; 1078; 1010; 976; 912; 858; 814; 763; 749; 725; 695; 602; 570; 516; 491; 461; **¹H**-**NMR** (300 MHz, Toluol-d₈): δ (ppm) = 7.62-7.43 (m, 2H, Ar-C**H**); 7.36-7.29 (m, 1H, Ar-C**H**); 7.28-7.08 (m, 2H, Ar-C**H**); 6.86-6.66 (m, 3H, Ar-C**H**); 6.58 (d, *J* = 2.1 Hz, 1H, Ar-C**H**); 4.82 (s, 2H, -OC**H₂**Ph); 2.27 (s, 3H, -C**H₃**); 2.12 (d, *J* = 2.4 Hz, 3H, -C**H₃**); 2.07 (s, 3H, -C**H₃**); 1.86 (s, 3H, - C**H₃**)**; ¹³C**-**NMR** (75 MHz, Toluol-d₈): δ (ppm) = 154.2; 153.2; 137.7; 136.0; 135.1; 134.6; 131.3; 131.2; 129.7; 129.0; 128.6; 128.3; 128.2; 128.1; 74.94; 30.30; 20.53; 20.18; 16.93; 16.31; **⁷⁷Se-NMR** (57 MHz, Toluol-d₈): δ (ppm) = 207.3; **HR-MS (ESI-TOF):** ber. für C₂₃H₂₅O₂Se ([M+H]⁺): 413.10155, gef.: 413.10109; ber. für C₂₃H₂₄O₂SeNa ([M+Na]⁺): 435.0835, gef.: 435.08378; **C₂₃H₂₄O₂Se** (412.09 g/mol).

Analog wurden 3,3',5,5'-Tetra-tert-butylbiphenyl-2,2'-diol, **1c** und Bis(3-tert-butyl-5-methyl-2-hydroxyphenyl)selen, **1b** mit Benzylbromid zu den Hydroxyl-geschütztes Strukture IX, IXa umgesetzt.

### Darstellung des Hydroxyl-geschützten Monophosphites

### Synthese von 2-((2-(Benzyloxy)-3,5-dimethylphenyl)selanyl)-4,6-dimethylphenyl-bis(2,4-di-tert-butylphenyl)phosphit Ia, 4

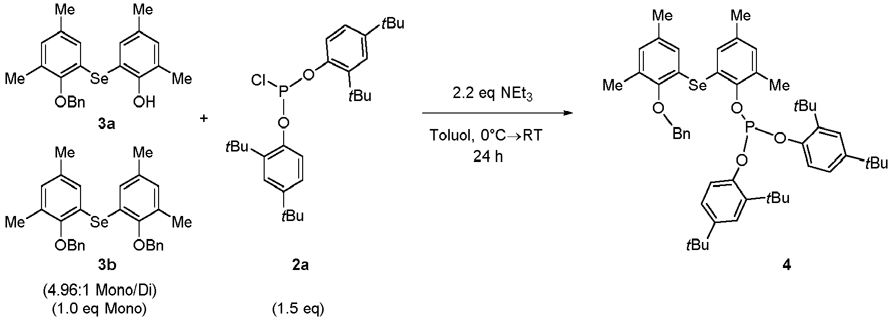

In einem ausgeheizten, unter Argon-Atmosphäre befindlichen 50 mL Schlenkgefäß wurden 547 mg (1.15 mmol, 1.5 eq, 86%ig) Bis(2,4-di-*tert*-butylphenyl)phosphorchloridit **2a** in 10 mL abs. Toluol gelöst und auf 0°C gekühlt. In einem separaten 10 mL Schlenkgefäß wurden 314 mg (0.762 mmol, 1.0 eq, Gemisch mit Verbindung Bis(2-(benzyloxy)-3,5-dimethylphenyl)selan **3b**, Verhältnis **3a/3b** 4.96:1) 2-((2-(benzyloxy)-3,5-dimethylphenyl)selanyl)-4,6-dimethylphenol **3** und 230 µL (170 mg, 1.68 mmol, 2.2 eq) Triethylamin in 10 mL abs. Toluol gelöst und zum vorgelegten Phosphorchloridit **2a** tropfenweise addiert. Hierbei wurde eine Gelbfärbung der Lösung mit gleichzeitiger Niederschlagsbildung beobachtet. Anschließend wurden weitere 5.0 mL abs. Toluol zugegeben und 30 Minuten bei 0°C sowie 16 Stunden bei RT gerührt. Das Reaktionsgemisch wurde zur vollständigen Abtrennung des entstandenen Niederschlages filtriert und der Feststoff mit 10 mL abs. Toluol gewaschen. Das Lösungsmittel wurde unter vermindertem Druck entfernt und das Rohprodukt säulenchromatographisch (100% H → 100:1 → 50:1 → 20:1 H/DCM → 100% DCM) aufgereinigt. Nach dreistündigem Trocknen des Produktes im Vakuum bei 50°C konnten 296 mg (0.347 mmol, 45%) der Titelverbindung **4** als farbloses Öl erhalten werden.

IR (ATR): *v̂* (cm⁻¹) = 2955; 2922; 2864; 1491; 1462; 1399; 1361; 1271; 1246; 1191; 1157; 1125; 1082; 1015; 924; 906; 886; 843; 816; 770; 748; 726; 687; 748; 726; 687; 663; 644; 600; 573; 494; ¹H-NMR (300 MHz, Toluol-d₈): δ (ppm) = 7.45 (d, *J* = 2.5 Hz, 2H, Ar-CH); 7.43-7.38 (m, 1H, Ar-CH); 7.34 (dd, *J* = 8.4 Hz, *J* = 2.1 Hz, 2H, Ar-CH); 7.26 (d, *J* = 2.5 Hz, 1H, Ar-CH); 7.09 (dt, *J* = 2.2 Hz, *J* = 1.1 Hz, 2H, Ar-CH); 7.03-6.95 (m, 2H, Ar-CH); 6.89-6.81 (m, 3H, Ar-CH); 6.72 (dd, *J* = 1.3 Hz, *J* = 0.7 Hz, 1H, Ar-CH); 6.62-6.57 (m, 1H, Ar-CH); 4.63 (s, 2H, -CH₂Ph); 2.20 (d, *J* = 0.9 Hz, 3H, 5-CH₃); 1.92 (d, *J* = 0.7 Hz, 3H, 5-CH₃); 1.77-1.75 (m, 3H, 3-CH₃);1.74 (d, *J* = 0.7 Hz, 3H, 3-CH₃); 1.29 (s, 18H, -C(CH₃)₃); 1.00 (s, 18H, -C(CH₃)₃); ⁷⁷Se-NMR (57 MHz, Toluol-d₈): δ (ppm) = 317.3 ppm (d, *J*_{Se-P} = 78.6 Hz); ³¹P-NMR (122 MHz, Toluol-d₈): 5 (ppm) = 137.0 (*J*_{P-Se} = 78.8 Hz); HR-MS (ESI-TOF): ber. für C₅₁H₆₆O₄PSe ([M+H]⁺): 853.38646, gef.: 853.38638; ber. für C₅₁H₆₅O₄PSeNa ([M+Na]⁺): 876.37140, gef.: 876.37119; ber. für C₅₁H₆₅O₄PSeK ([M+K]⁺): 891.34232, gef.: 891.34248; C₅₁H₆₅O₄PSe (852.38 g/mol).

### Hydroformylierung

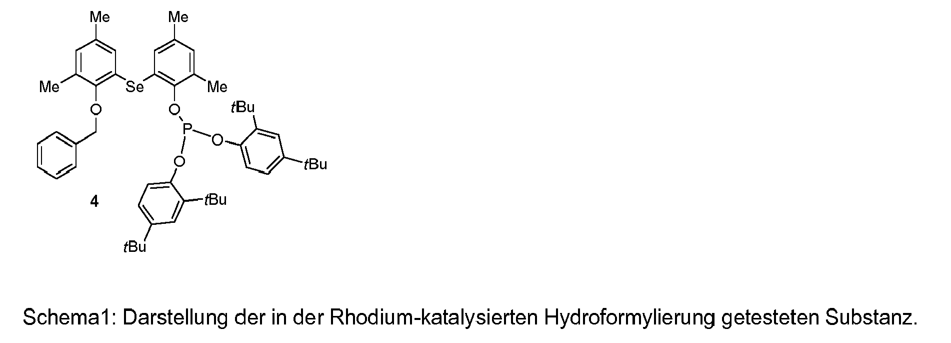

**Tabelle 2: Darstellung der Katalyse-Experimente unter der Verwendung von Organoselenverbindungen.**

| Eintrag | Ligand | Olefin /LM | Verhältnis Rh/ Ligand/Olefin | p [bar] | T [°C] | t [h] | S [%] |
|---|---|---|---|---|---|---|---|
| 1 | 4* | n-Octen / Toluol | 1:5:2230 (100 ppm Rh) | 50 | 120 | 4 | 20.2 |

| | | | | | | | |
|---|---|---|---|---|---|---|---|
| Erläuterungen zu Tabelle 1: p = Druck, T = Temperatur, t = Zeit, A = Ausbeute; S = n-Regioselektivität.*erfindungsgemäss | | | | | | | |

Bei Verwendung des erfindungsgemässen Liganden 4 konnte eine gute Selektivität bei der Hydroformylierung von n-Octenen erreicht werden.

Somit sind die Hydroxyl-Gruppe geschützte Selena-Monophosphite als Liganden in der Hydroformylierung einsetzbar.

## Patentansprüche

1. Verbindung eines heterozyklischen Selena-Phosphites, welches eine allgemeine Struktur (I) aufweist 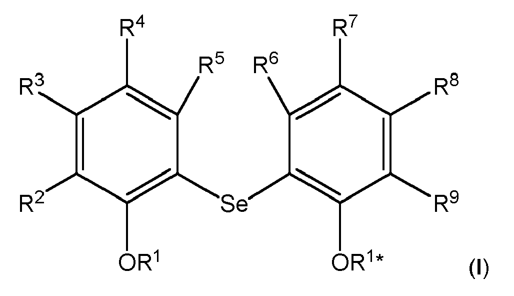 wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei die Gruppe -R¹ ausgewählt ist aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₁-C₁₂)Alkyl, -(C₆-C₂₀)-Aryl-O-(C₆-C₂₀)-Aryl, -(C=O)-O-(C₁-C₁₂)-Alkyl, -acetyl,
wobei die Alkylgruppen linear, verzweigt oder cyclisch sind, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
und die Gruppe -R^{1*} einer organofunktionellen Phosphit-Gruppe entspricht.

2. Verbindung nach Anspruch 1, **dadurch gekennzeichnet, dass** im heterozyklischen Selena-Phosphit der allgemeinen Struktur (**I**) -R^{1*} einer organofunktionellen Phosphit-Gruppe ausgewählt aus den Strukturen (**II**), (**III**), (**IV**), (**V**), (**VI**) und (**VII**) entspricht, 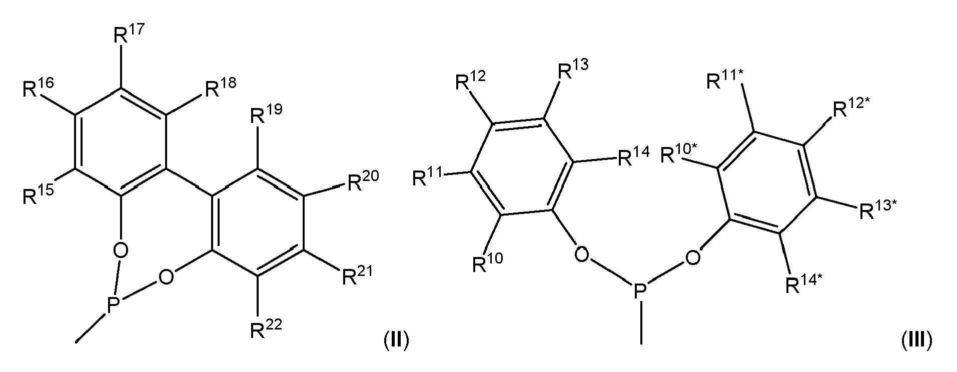 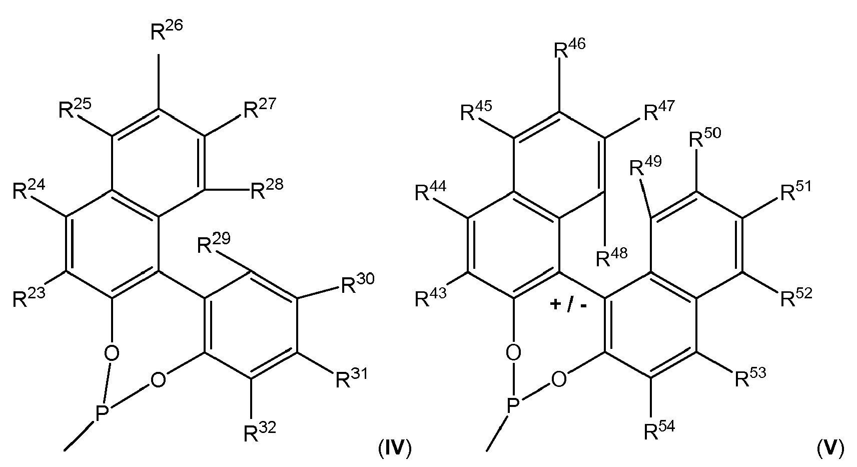 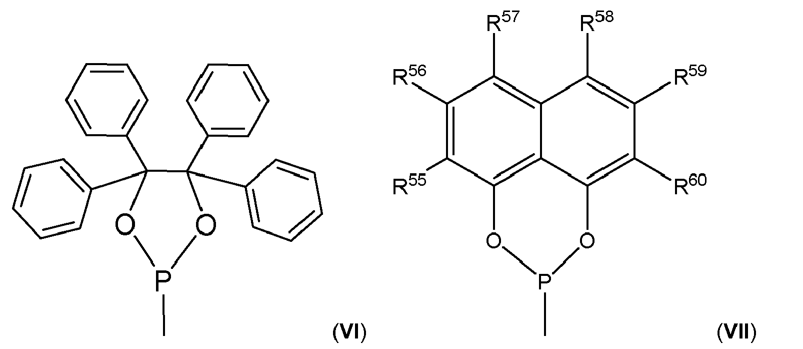 wobei die Reste
R¹⁵, R¹⁶, R¹⁷, R¹⁸, R¹⁹, R²⁰, R²¹, R²²-in Struktur (**II**), R¹⁰, R¹¹, R¹², R¹³, R¹⁴, R^{10*}, R^{11*}, R^{12*}, R^{13*}, R^{14*} in der Struktur (**III**), R²³, R²⁴, R²⁵, R²⁶, R²⁷, R²⁸, R²⁹, R³⁰, R³¹ und R³² in Struktur (**IV**), R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur (**V**), R⁵⁵, R⁵⁶, R⁵⁷, R⁵⁸, R⁵⁹ und R⁶⁰ in Struktur (**VII**), jeweils in der jeweiligen Struktur unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und die jeweilige substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

3. Verbindung nach Anspruch 1, **dadurch gekennzeichnet, dass**
die Gruppen -R¹ in der Struktur (I) ausgewählt ist aus: -(C₁-C₁₂)-Alkyl, -(C₁-C₁₂)-Alkyl-O-(C₁-C₁₂)-Alkyl, -(C₆-C₂₀)-Aryl, -(C₆-C₂₀)-Aryl-O-(C₁-C₁₂)Alkyl, -(C₁-C₁₂)-Alkyl-O-(C₆-C₂₀)-Aryl, -(C₆-C₂₀)-Aryl-O-(C₆-C₂₀)-Aryl, -(C=O)-O-(C₁-C₁₂)-Alkyl, wobei die Alkylgruppen linear, verzweigt oder cyclisch sind, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl und/oder -(C₆-C₂₀)-Aryl.

4. Verbindung nach einem der Ansprüche 1 bis 3, **dadurch gekennzeichnet, dass** das heterozyklische Selena-Phosphit der allgemeinen Struktur (I) ausgewählt ist aus mindestens einer Verbindung der Struktur (la) 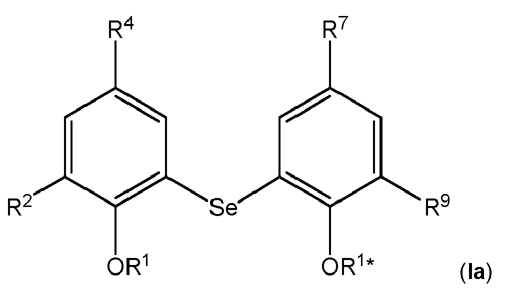 wobei R², R⁴, R⁷ und R⁹ jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sind,
wobei die Gruppe -R¹ ausgewählt ist aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₁-C₁₂)Alkyl, -(C₆-C₂₀)-Aryl-O-(C₆-C₂₀)-Aryl, wobei die Alkylgruppen linear, verzweigt oder cyclisch sind, und die Gruppe -R^{1*} in Struktur (**Ia**) einer organofunktionellen Phosphit-Gruppe entspricht.

5. Verbindung nach einem der Ansprüche 1 bis 4, **dadurch gekennzeichnet, dass** das -R^{1*} im heterozyklischen Selena-Phosphit der allgemeinen Struktur (**I**) oder (**Ia**) ausgewählt aus den Strukturen (**II**), (**III**), (**IV**), (**V**), (**VI**), und (**VII**) entspricht 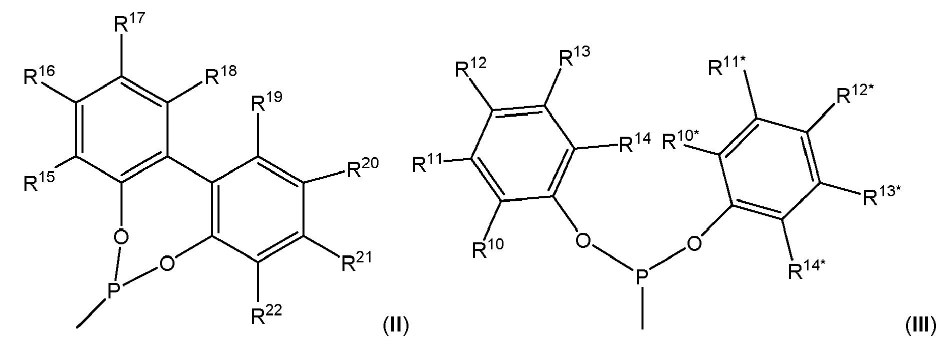 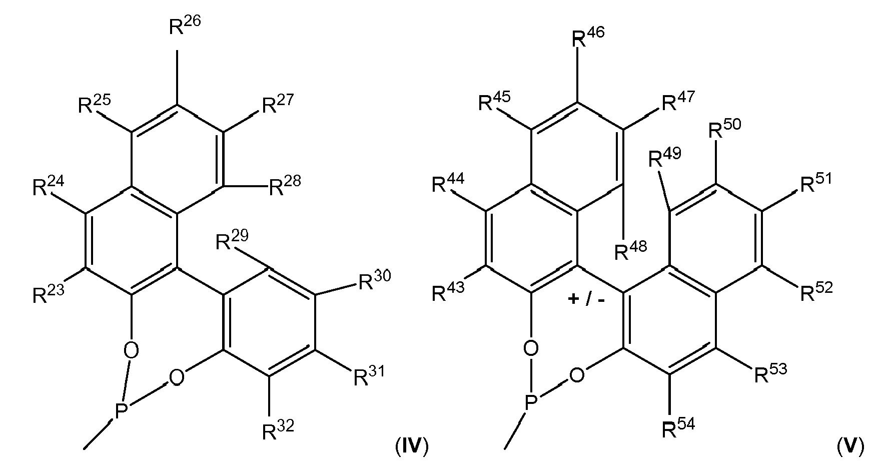 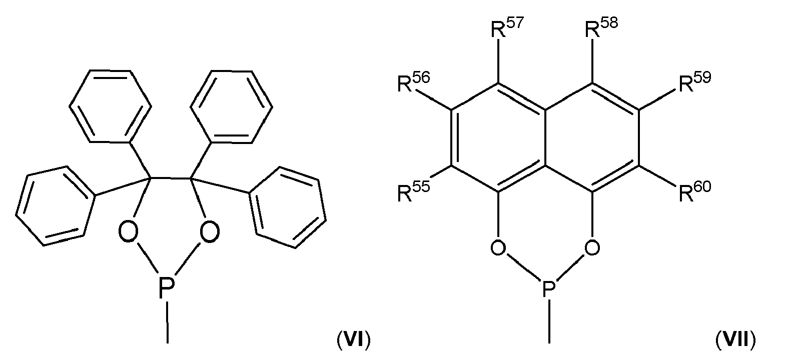 wobei die Reste
R¹⁵, R¹⁶, R¹⁷, R¹⁸, R¹⁹, R²⁰, R²¹, R²² in Struktur (**II**), R¹⁰, R¹¹, R¹², R¹³, R¹⁴, R^{10*}, R^{11*}, R^{12*}, R^{13*}, R^{14*} in der Struktur (**III**),
R²³, R²⁴, R²⁵, R²⁶, R²⁷, R²⁶, R²⁹, R³⁰, R³¹ und R³² in Struktur (**IV**), R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur (**V**), R⁵⁵, R⁵⁶, R⁵⁷, R⁵⁸, R⁵⁹ und R⁶⁰ in Struktur (**VII**), jeweils für jede Struktur unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen wobei die Alkylgruppen linear, verzweigt oder cyclisch sind.

6. Verbindung nach einem der Ansprüche 1 bis 5, **dadurch gekennzeichnet, dass** das -R^{1*} im heterozyklischen Selena-Phosphit der allgemeinen Struktur (**I**) oder (**Ia**) ausgewählt ist aus Struktur (**III**),
und die Gruppe -R¹ ausgewählt ist aus -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₁-C₁₂)Alkyl, -(C₆-C₂₀)-Aryl-O-(C₆-C₂₀)-Aryl, wobei die Alkylgruppen linear, verzweigt oder cyclisch sind, 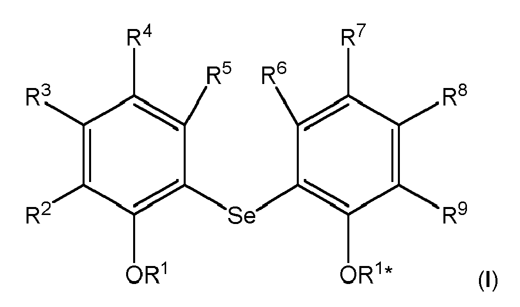 wobei R², R³, R⁴, R⁵, R⁶, R⁷, R⁸ und R⁹ in Struktur (**I**) jeweils unabhängig voneinander ausgewählt sind aus: -H, -(C₁-C₁₂)-Alkyl, -O-(C₁-C₁₂)-Alkyl, wobei die Alkylgruppen linear, verzweigt oder cyclisch sind, oder 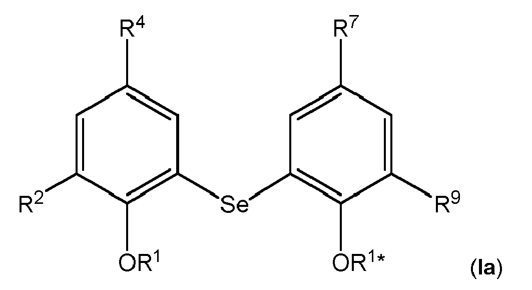 wobei R², R⁴, R⁷ und R⁹ in Struktur (**Ia**) jeweils unabhängig voneinander ausgewählt sind aus - (C₁-C₁₂)-Alkyl, -O-(C₁-C₁₂)-Alkyl, wobei die Alkylgruppen linear, verzweigt oder cyclisch sind, und 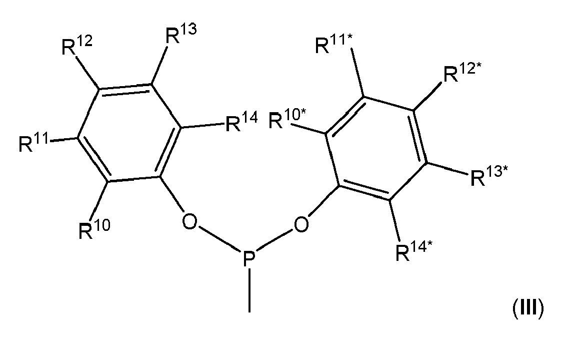 mit R¹⁰, R¹¹, R¹², R¹³, R¹⁴, R^{10*}, R^{11*}, R^{12*}, R^{13*}, R^{14*} in der Struktur (**III**), jeweils unabhängig voneinander ausgewählt aus: -H, -(C₁-C₁₂)-Alkyl, -O-(C₁-C₁₂)-Alkyl, wobei die Alkylgruppen linear, verzweigt oder cyclisch sind.

7. Verfahren zur Herstellung mindestens eines heterozyklischen Selena-Phosphites der allgemeinen Struktur (I) 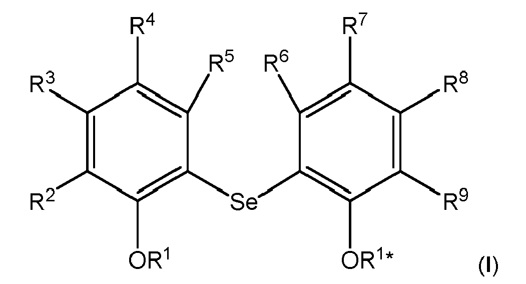 wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und
wobei die Gruppe -R¹ ausgewählt ist aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₁-C₁₂)Alkyl, -(C₆-C₂₀)-Aryl-O-(C₆-C₂₀)-Aryl, -C=O-O-(C₁-C₁₂)-Alkyl, acetyl, wobei die Alkylgruppen linear, verzweigt oder cyclisch sind, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl, und die Gruppe -R^{1*} einer organofunktionellen Phosphit-Gruppe entspricht.
umfassend mindestens den Verfahrensschritt
(i) Umsetzen eines Selenodiaryls der allgemeinen Struktur (IX) 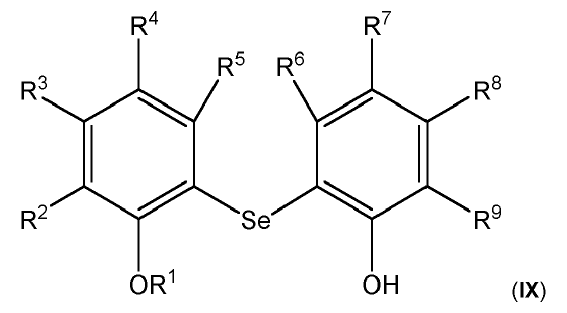 wobei R², R³, R⁴, R⁵, R⁶, R⁷, R⁸ und R⁹ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, -OC=O-(C₁-C₁₂)-Alkyl, -S-Alkyl, -S-Aryl, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -SO₃H, -CN, -N[(C₁-C₁₂)-Alkyl]₂, wobei die Alkylgruppen linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und substituierte -(C₆-C₂₀)-Arylgruppe, mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
und Gruppe -R¹ ausgewählt ist aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₁-C₁₂)Alkyl, -(C₆-C₂₀)-Aryl-O-(C₆-C₂₀)-Aryl, -C=O-O-(C₁-C₁₂)-Alkyl, -acetyl, wobei die Alkylgruppen linear, verzweigt oder cyclisch sind, wobei die genannten Alkyl- und Arylgruppen jeweils unabhängig unsubstituiert oder substituiert sind, wobei substituierte -(C₁-C₁₂)-Alkylgruppen und substituierte -(C₆-C₂₀)-Arylgruppen mindestens einen Substituenten aufweisen und der mindestens eine Substituent jeweils unabhängig ausgewählt ist aus - (C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl,
(ii) mit mindestens einer Halogenphosphit-Verbindung R^{1*} Hal der Formel (**X**), wobei Hai jeweils unabhängig ausgewählt ist aus Fluor, Chlor, Brom, Jod, und wobei -R^{1*} einer organofunktionellen bivalenten Phosphit-Gruppe entspricht,
(iii) und erhalten mindestens eines heterozyklischen Selena-Phosphits der allgemeinen Struktur (**I**).

8. Verfahren nach Anspruch 7, **dadurch gekennzeichnet, dass** in den Halogenphosphit-Verbindungen R^{1*} Hal der Formel (**X**) Hal jeweils unabhängig ausgewählt ist aus Fluor, Chlor, Brom, Jod, und -R^{1*} jeweils ausgewählt ist aus den Strukturen (**II**), (**III**), (**IV**), (**V**), (**VI**) und (**VII**) 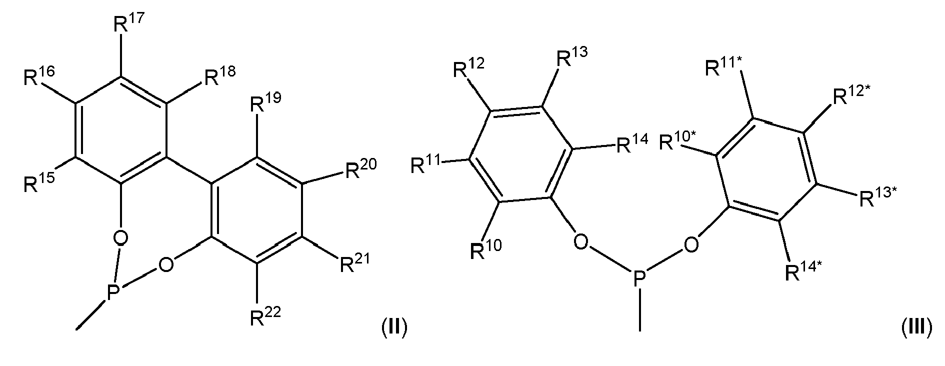 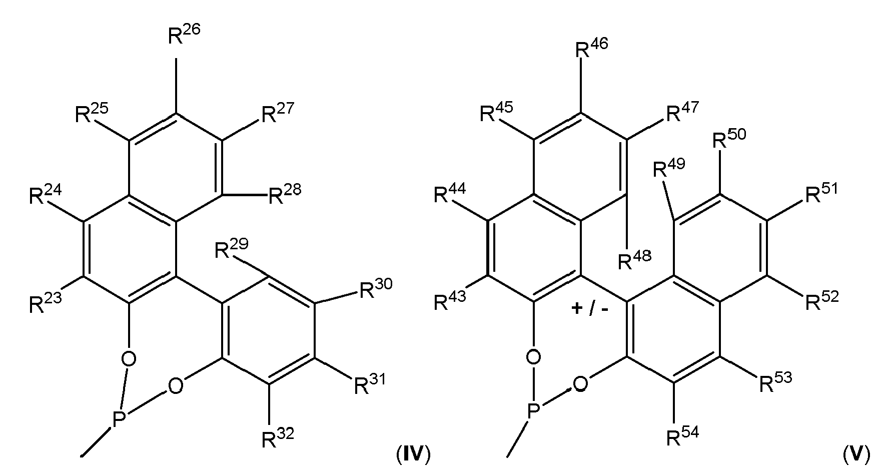 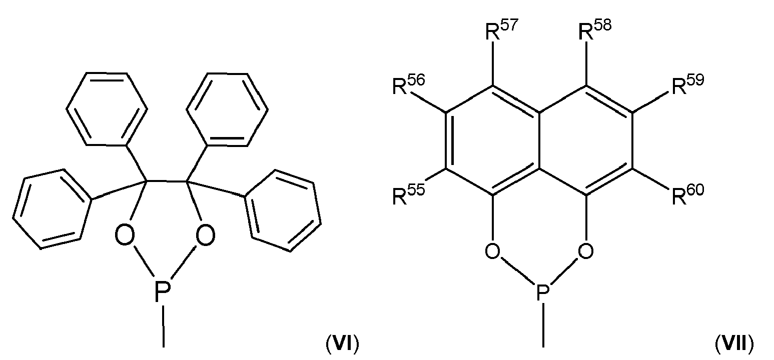 wobei die Reste
R¹⁵, R¹⁶, R¹⁷, R¹⁸, R¹⁹, R²⁰, R²¹, R²² -in Struktur (**II**), R¹⁰, R¹¹, R¹², R¹³, R¹⁴, R^{10*}, R^{11*}, R^{12*}, R^{13*}, R^{14*} in der Struktur (**III**), R²³, R²⁴, R²⁵, R²⁶, R²⁷, R²⁶, R²⁹, R³⁰, R³¹ und R³² in Struktur (**IV**), R⁴³, R⁴⁴, R⁴⁵, R⁴⁶, R⁴⁷, R⁴⁸, R⁴⁹, R⁵⁰, R⁵¹, R⁵², R⁵³ und R⁵⁴ in Struktur (**V**), R⁵⁵, R⁵⁶, R⁵⁷, R⁵⁸, R⁵⁹ und R⁶⁰ in Struktur (**VII**),
jeweils unabhängig in der jeweiligen Struktur voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sind, wobei die Alkyl- und Arylgruppen jeweils unabhängig unsubstituiert oder substituiert sind, wobei die jeweilige substituierte -(C₁-C₁₂)-Alkylgruppe und die jeweilige substituierte -(C₆-C₂₀)-Arylgruppe mindestens einen Substituenten aufweist und der mindestens eine Substituent jeweils unabhängig ausgewählt ist aus -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

9. Verfahren nach Anspruch 7 oder 8, **dadurch gekennzeichnet, dass** in der Halogenphosphit-Verbindung R^{1*} Hal der Formel (**X**) Hal ausgewählt ist aus Chlor oder Brom, und
-R^{1*} ausgewählt ist aus den Strukturen (**II**), (**III**), (**IV**), (**V**), (**VI**) und (**VII**) 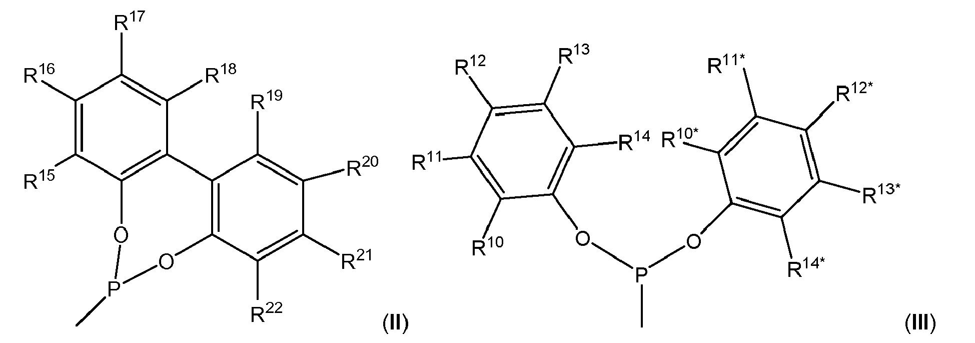 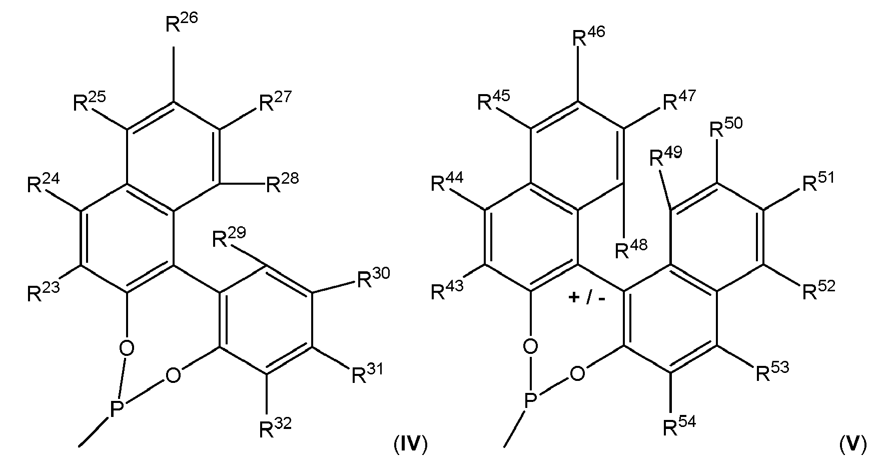 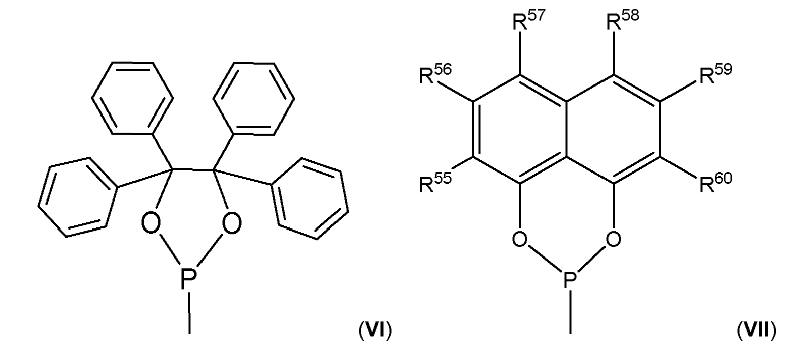 wobei die Reste
R¹⁵, R¹⁶, R¹⁷, R¹⁸, R¹⁹, R²⁰, R²¹, R²²-in Struktur (**II**), R¹⁰, R¹¹, R¹², R¹³, R¹⁴, R^{10*}, R^{11*}, R^{12*}, R^{13*}, R^{14*} in der Struktur (**III**), R²³, R²⁴, R²⁵, R²⁶, R²⁷, R²⁸, R²⁹, R³⁰, R³¹ und R³² in Struktur (**IV**), R⁴³, R⁴⁴, R⁴⁵, R⁴⁶, R⁴⁷, R⁴⁸, R⁴⁹, R⁵⁰, R⁵¹, R⁵², R⁵³ Und R⁵⁴ in Struktur (**V**), R⁵⁵, R⁵⁶, R⁵⁷, R⁵⁸, R⁵⁹ und R⁶¹ in Struktur (**VII**), jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sind.

10. Verfahren nach einem der Ansprüche 7 bis 9, **dadurch gekennzeichnet, dass** das Selenodiaryl der allgemeinen Struktur (**IXa**) entspricht 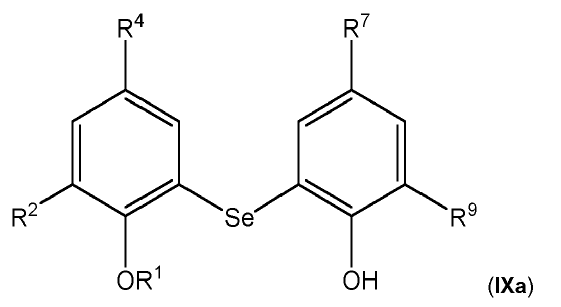 wobei R², R⁴, R⁷ und R⁹ jeweils unabhängig voneinander ausgewählt sind aus: -(C₁-C₁₂)-Alkyl, -O-(C₁-C₁₂)-Alkyl, -(C₆-C₂₀)-Aryl, -O-(C₆-C₂₀)-Aryl, -Halogen, wobei die Alkylgruppen linear, verzweigt oder cyclisch sind,
wobei die Gruppe -R¹ ausgewählt ist aus: -(C₁-C₁₂)-Alkyl, -(C₁-C₁₂)-Alkyl-O-(C₁-C₁₂)-Alkyl, -(C₆-C₂₀)-Aryl, -(C₁-C₁₂)-Alkyl-O-(C₆-C₂₀)-Aryl, -(C₆-C₂₀)-Aryl-O-(C₁-C₁₂)Alkyl, -(C₆-C₂₀)-Aryl-O-(C₆-C₂₀)-Aryl, wobei die Alkylgruppen linear, verzweigt oder cyclisch sind.

11. Verfahren nach einem der Ansprüche 7 bis 10,
wobei (i) die Umsetzung in Gegenwart einer Base, insbesondere eines Amins oder einer Pyridinbase, vorzugsweise eines Alkylamins, wie Triethylamin oder Dimethylaminobutan, besonders bevorzugt Triethylamin.

12. Verfahren nach einem der Ansprüche 7 bis 11, **dadurch gekennzeichnet,**
**dass** das Selenodiaryl der allgemeinen Struktur (**IX**) oder (**IXa**) mit R^{1*} Hal der Formel (**X**) im molaren Verhältnis von 10 : 1 bis 1 : 10 umgesetzt wird, vorzugsweise im Verhältnis von 4: 1 bis 1 : 4.

13. Verfahren nach einem der Ansprüche 7 bis 12,
wobei (i) die Umsetzung im Temperaturbereich von -45 bis 80 °C erfolgt, insbesondere von - 15 bis 30°C.

14. Verfahren nach einem der Ansprüche 7 bis 13,
wobei (i) die Umsetzung in einem aprotischen Lösemittel erfolgt, insbesondere ist das Lösemittel ausgewählt aus organischen aromatischen, halogenierten Lösemitteln oder Kohlenwasserstoffen.

15. Verwendung eines heterozyklischen Selena-Phosphits nach einem der Ansprüche 1 bis 6 oder erhalten nach einem der Ansprüche 7 bis 14 als Ligand.
